# EUROPEAN PATENT SPECIFICATION

(11) **EP 1 315 468 B1**
(45) Date of publication and mention of the grant of the patent: **01.06.2005**
(21) Application number: 01971904.6
(22) Date of filing: 10.08.2001
(51) Int. Cl.: A61F 2/00

(54) **FLEXIBLE IMPLANT**
BIEGSAMES IMPLANTAT
IMPLANT SOUPLE

(30) Priority: 04.09.2000 DE 10043396
(43) Date of publication of application: 04.06.2003
(73) Proprietor: Ethicon GmbH, 22851 Norderstedt (DE)
(72) Inventor: SCHULDT-HEMPE, Barbara, 24576 Bad Bramstedt (DE); WALTHER, Christoph, 24568 Kattendorf (DE)
(74) Representative: UEXKÜLL & STOLBERG
(86) International application number: PCT/EP2001/009299
(87) International publication number: WO 2002/019944

(56) References cited:
- EP-A- 0 797 962

## Description

The invention relates to a flexible implant with a mesh-like basic structure.

Flexible implants with a mesh-like basic structure are widespread, e.g., in the form of implant meshes. They are inserted into the patient's body during a surgical procedure, e.g. at the abdominal wall, in order to reinforce the body tissue at least temporarily and to prevent complications such as e.g. hernias.

Such implant meshes are frequently produced from monofilaments, e.g., from polyethylene and in particular from polypropylene. US 3 124 136 A shows an implant mesh of polyethylene with a pore size of less than 1 mm. In US 4 452 245 A a double-laid implant of polypropylene monofilaments is described in which the pores of the mesh structure are greater than 1 mm.

As a rule, commercial implant meshes of monofilaments have small pores and are relatively rigid. This causes a permanent mechanical stimulus and an irritation of the tissue at the site of the surgery and often leads to the formation of cicatricial layers.

There are also commercial implant meshes of multifilament yarns, e.g. polyester or polypropylene. Resorbable materials can be used here. Thus, e.g., partly resorbable meshes are known in which, apart from polypropylene, polyglactin 910, a copolymerisate of 9 parts glycolide and 1 part lactide, is used.

EP-A-0797962 discloses an implant with a flexible basic structure made from a knitted fabric of multifilaments which fabric has meshes with an inside width in the range from 1 mm to 8 mm.

Although implant meshes of multifilament yarns are relatively soft, the multifilament yarns have a large surface and show a capillary effect, which can lead to undesired tissue reactions and is associated with a danger of infection for the patient.

The object of the invention is to provide a flexible implant which is tissue-friendly and relatively soft and behaves largely inertly.

This object is achieved by a flexible implant with the features of claim 1. Advantageous designs of the invention result from the dependent claims.

The flexible implant according to the invention contains a mesh-like basic structure of monofilaments, the diameter of which is in the range from 0.02 mm to 0.30 mm (preferably in the range from 0.05 mm to 0.30 mm) and the bending elasticity modulus of which is smaller than 3500 N/mm². The basic structure contains pores the size of which lies in the range from 1.5 mm to 8.0 mm over more than 90% of the total area of the pores. The bending force of the basic structure, measured in a three-point bending test with a span of 11 mm and normalized to a sample width of 10 mm, is at most 30 mN.

The mesh-like basic structure is preferably the only component of the implant according to the invention. Additional components can also be provided, however, e.g. a coating of the basic structure or additional threads inserted into the basic structure with other properties than the monofilaments of the basic structure have.

The mesh-like basic structure of the implant according to the invention consists of monofilaments. Thus the surface which can come into contact with the tissue or the tissue liquids is relatively small. There is no capillary effect and the danger of infection is minimal. The monofilaments, which as a rule have a smooth surface, behave essentially inertly. The implant according to the invention thus has the good properties of implant meshes which are produced from monofilaments.

Surprisingly it turned out that given the choice of parameters mentioned above for the monofilaments and the basic structure, despite the use of monofilaments, a soft implant can be made which causes, at most, slight mechanical irritation of the tissue (e.g. of the peritoneum) and causes no, or only slight, adhesions. The implant according to the invention thus also has the advantages of the implant meshes of multifilaments.

The bending elasticity modulus of the monofilaments is a material property which corresponds to the Young's modulus (measured in a bending test). With the plastics used for monofilaments, the bending elasticity modulus depends inter alia somewhat on the production conditions (in particular the stretching conditions and the thermal post-treatments) and dimensions of the monofilaments. The value of 3500 N/mm² is lower than the bending elasticity modulus of usual polypropylene monofilaments for mesh production, which is of the order of 5000 to 9000 N/mm². The monofilaments of the mesh-like basic structure are thus relatively soft and have a high flexibility with a diameter in the range from 0.02 mm to 0.30 mm.

The predominant share of the pores (i.e. the proportion of the pores which accounts for more than 90% of the total area of the pores in the basic structure) has a size in the range from 1.5 mm to 8.0 mm, and is therefore relatively large. This contributes to a minimisation of the mechanical tissue irritation originating in the implant. Individual pores of the basic structure may (e.g. for production reasons) be smaller than 1.5 mm, but this has no noteworthy effect on the overall properties of the implant.

The flexibility or softness of the implant depends not only on the monofilaments used as material and the size of the pores as such, but e.g. also on the weft-knitted or warp-knitted structure of the basic structure and the matching of the parameters to each other. Thus an upper limit for the bending force of the basic structure is stated as a further parameter which characterizes the implant according to the invention. This bending force emerges from the evaluation of a force-path diagram in which the deflection (i.e. the path) of a sample of the basic structure is plotted as a function of the force acting on the sample. For this, a sample of the basic structure is placed on two supports, 11 mm apart, the force acting perpendicular to the sample and in the middle between the two supports. The deflection increases as the force grows, until the force reaches a maximum and falls again as the deflection continues to increase. This maximum force is called bending force and is normalized to a sample width of 10 mm. The bending force is therefore a measure of the elasticity behaviour of the implant as a whole.

Suitable as materials for the monofilaments of the basic structure are, e.g., thermoplastically workable fluorinated homopolymers, thermoplastically workable fluorinated copolymers, polyolefins and mixtures of such materials.

The basic structure preferably contains monofilaments of polyvinylidene fluoride, copolymers of vinylidene fluoride and hexafluoropropene and/or mixtures of such materials. Mixtures of polyvinylidene fluoride (PVDF; thus a homopolymer of vinylidene fluoride) and a copolymer of 95 wt.-% vinylidene fluoride and 5 wt.-% hexafluoropropene are particularly suitable. The mixture ratio of the homopolymer to the copolymer can be, e.g., 50 : 50 or 80 : 20; the weight ratio is stated each time. Such polymers can be coloured in the melt, e.g. with the blue colour pigment copper phthalocyanine blue ("Color Index" CI. No. 74160). Monofilaments with a bending elasticity modulus of approx. 1200 N/mm² to approx. 2400 N/mm² can be produced from such mixtures.

If polyolefins are used as monofilaments, polyethylene or polypropylene for example are suitable according to selected production conditions.

The basic structure can also contain monofilaments of a resorbable material, e.g. of poly-p-dioxanone or of a copolymer of glycolides and caprolactones. Such a copolymer is marketed for example by Ethicon under the name "Monocryl". If resorbable monofilaments are used, the basic structure can be made completely, but also only partly, from resorbable material.

Preferably, the tensile strength of at least a part of the monofilaments is at least 400 N/mm². Such tensile strengths can be achieved, e.g., with the above-mentioned mixtures of polyvinylidene fluoride and a copolymer of vinylidene fluoride and hexafluoropropene.

The basic structure preferably contains a weft-knitted product or warp-knitted product, warp-knitted products preferably being made as crochet galloon ware. Weft-knitted products and warp-knitted products have proved suitable for flexible implants and can be made in a plurality of patterns.

In the following the invention is explained in detail by means of embodiments. The drawings show in
- Figure 1: the typical shape of a force-path diagram which is measured in a three-point bending test on a monofilament or on the basic structure of a flexible implant,
- Figure 2: a schematic illustration of the warp-knitted structure in the case of the implant according to the invention according to example 1,
- Figure 3: a schematic illustration of the warp-knitted structure in the case of the implant according to the invention according to examples 2 and 3 and
- Figure 4: a schematic illustration of the warp-knitted structure in the case of the implant according to the invention according to examples 4 and 5.

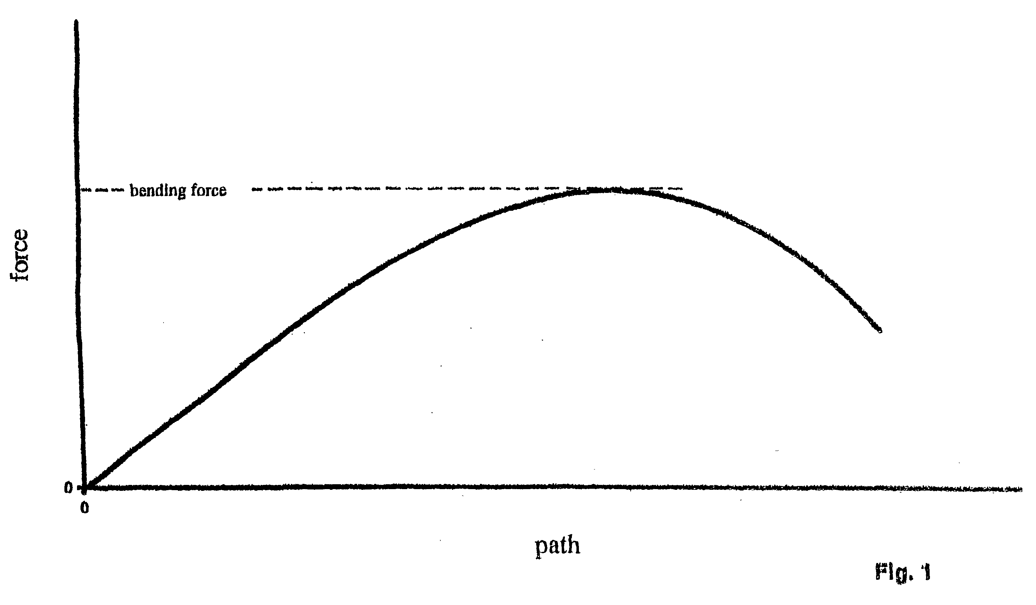

The shape of a typical force-path diagram is shown in Figure 1 as results when carrying out a three-point bending test on a monofilament or on the mesh-like basic structure of a flexible implant.

The properties listed below of monofilaments can be established in such a three-point bending test. A force is exerted on a monofilament supported at two points in the middle between the two support points and perpendicular to the line connecting of the support points. This force and the deflection of the monofilament ("path") caused by it are measured. As long as the deflection is small, the force grows linearly with the deflection, but more slowly thereafter. After a maximum force is reached, the force drops while the deflection continues to increase.

The force maximum in the force-path diagram of a given sample is called the bending force of the sample. The bending force is a measure of the flexibility behaviour of the sample, thus the monofilament here. The greater the bending force, the more rigid the monofilament. The bending force is given in N and depends on the test conditions, essentially on the distance between the two support points (span). In the three-point bending tests which led to the numerical values given below for the monofilaments, the span was 4 mm. The test speed, i.e. the speed at which the deflection of the sample is increased, was 50 mm/minute.

The bending rigidity of the sample can also be measured from the force-path diagram. The bending rigidity is the slope in the linear range of the measurement curve. The unit of measurement is N/mm.

The bending elasticity modulus of a monofilament can be calculated from a force-path diagram, taking the diameter of the monofilament into account. The bending elasticity modulus corresponds to the Young's modulus and is given in N/mm². This variable is largely independent of the test conditions, but depends not only on the material as such (like, e.g., in the case of metals), but also somewhat on the production conditions and the dimensions of the monofilaments.

A three-point bending test can be carried out in an analogous manner on an areal flexible implant or on its mesh-like basic structure. The sample is supported along two lines which run parallel to each other the distance of the span apart.

For the tests in which the parameters stated below of the mesh-like basic structure were established according to the embodiments, the span was 11 mm and the test speed (as for the monofilaments) was 50 mm/minute. In order to minimise edge effects, samples with a width of at least 25 mm were used.

As with the three-point bending tests on monofilaments, the bending rigidity (in N/mm) of the mesh-like basic structure results from the force-path diagram as the slope in the linear range of the curve. In order to be able to compare samples of different widths, a normalisation to the width of the sample took place. The numerical values given below relate to a sample width of 10 mm. If for example the sample width was actually 25 mm, this standardization took place by dividing the slope read off directly from the force-path diagram by 2.5.

The bending force is obtained by evaluation of the force-path diagram as force maximum of the curve. The numerical values given below were standardized to a sample width of 10 mm. The bending force is a measure of the rigidity of the implant or of the mesh-like basic structure as a whole. The greater the bending force, the more rigid and less flexible the implant.

Again, the bending elasticity modulus can be calculated.

Tables 1, 2 and 3 show respectively, for monofilaments of various materials and with various diameters, the bending elasticity modulus, the bending rigidity and the bending force. These variables were each established using three-point bending tests, as explained.

"PDS" is an abbreviation for poly-p-dioxanone, a resorbable material.

"Monocryl" is an Ethicon trade name for a resorbable copolymer of glycolides and caprolactones.

"Pronova" is an Ethicon trade name for monofilaments of a mixture of polyvinylidene fluoride (PVDF) and a copolymer of 95 wt.-% vinylidene fluoride and 5 wt.-% hexafluoropropene. Polyvinylidene fluoride and the copolymer are marketed for example by Solvay Advanced Polymers under the trade name "Solef 1008" and "Solef 11010", respectively. Surgical filaments of such materials are described in US 4 564 013 A. In the case of the monofilaments of "Pronova" listed in Tables 1 to 3, the mixture ratio of the homopolymer to the copolymer is 50 : 50 (in wt.-%).

**Table 1**

| **Bending elasticity modulus of various monofilaments** | | | | |
|---|---|---|---|---|
| **Diameter** | **Bending elasticity modulus** | | | |
| **[mm]** | | **[N/mm**^{**2**}**]** | | |
| | **PP** | **PDS** | **Monocryl** | **Pronova** |
| 0.126 | 6691 | | | |
| 0.086 | 8905 | | | |
| 0.18 | 5097 | | | |
| 0.23 | 4970 | | | |
| | | | | |
| 0.224 | | 1260 | | |
| 0.173 | | 1575 | | |
| 0.106 | | 1201 | | |
| | | | | |
| 0.232 | | | 537 | |
| 0.16 | | | 971 | |
| 0.132 | | | 783 | |
| | | | | |
| 0.248 | | | | 1944 |
| 0.199 | | | | 2256 |
| 0.136 | | | | 2118 |
| 0.093 | | | | 1232 |

**Table 2**

| **Bending rigidity of various monofilaments** | | | | |
|---|---|---|---|---|
| **Diameter** | **Bending rigidity** | | | |
| **[mm]** | **[N/mm]** | | | |
| | **PP** | **PDS** | **Monocryl** | **Pronova** |
| 0.126 | 0.065 | | | |
| 0.086 | 0.024 | | | |
| 0.18 | 0.197 | | | |
| 0.23 | 0.152 | | | |
| | | | | |
| 0.224 | | 0.1213 | | |
| 0.173 | | 0.0473 | | |
| 0.106 | | 0.0057 | | |
| | | | | |
| 0.232 | | | 0.0573 | |
| 0.16 | | | 0.0233 | |
| 0.132 | | | 0.0087 | |
| | | | | |
| 0.248 | | | | 0.274 |
| 0.199 | | | | 0.1247 |
| 0.136 | | | | 0.027 |
| 0.093 | | | | 0.0037 |

**Table 3**

| **Bending force of various monofilaments** | | | | |
|---|---|---|---|---|
| **Diameter** | **Bending force** | | | |
| **[mm]** | **[mN]** | | | |
| | **PP** | **PDS** | **Monocryl** | **Pronova** |
| 0.126 | 23 | | | |
| 0.086 | 10 | | | |
| 0.18 | 66 | | | |
| 0.23 | 157 | | | |
| | | | | |
| 0.224 | | 71 | | |
| 0.173 | | 30.5 | | |
| 0.106 | | 5.9 | | |
| | | | | |
| 0.232 | | | 36.1 | |
| 0.16 | | | 14.1 | |
| 0.132 | | | 6.21 | |
| | | | | |
| 0.248 | | | | 116.7 |
| 0.199 | | | | 57.7 |
| 0.136 | | | | 14.3 |
| 0.093 | | | | 4.31 |

**Table 4**

| **Properties of the implants according to Examples 1 to 5 and of two conventional implants of polypropylene (PP)** | | | | |
|---|---|---|---|---|
| **Implant** | **Pore size** | **Bending force** | **Bending rigidity** | **Bending elasticity modulus** |
| | | **[mN]** | **[N/mm]** | **[N/mm**^{**2**}**]** |
| Example 1 | ca. 6.5 | 2.2 | 0.00054 | 0.83 |
| Example 2 | ca. 3.5 | 4.4 | 0.00141 | 2.13 |
| Example 3 | ca. 5 | 4.5 | 0.00165 | 0.7 |
| Example 4 | ca. 2 | 5.2 | 0.00082 | 0.65 |
| Example 5 | ca. 3 | 5.3 | 0.00213 | 0.98 |
| PP ("Marlex") | ca. 0.8 | 116 | 0.01026 | 1.57 |
| PP ("Atrium") | ca. 1 | 33 | 0.00332 | 2.2 |

The monofilaments of PDS, "Monocryl" and "Pronova" are suitable for building up the mesh-like basic structure of an implant according to the invention. For comparison, the numerical values for monofilaments of various thicknesses of polypropylene (PP) are given in Tables 1 to 3. The bending elasticity modulus of polypropylene is greater than 4000 N/mm².

While the properties of the monofilaments are largely determined by the diameter and the bending elasticity modulus, thus by the numerical values in Table 1, Tables 2 and 3 illustrate the bending rigidity and the bending force which result directly from the force-path diagrams established in the three-point bending test. For monofilaments which are suitable for the flexible implant according to the invention, the bending rigidity should ,preferably be approx 0.08 N/mm at most for a diameter of 0.15 mm and approx 0.5 N/mm at most for a diameter of 0.25 mm. The bending force should preferably be smaller than approx 30 mN for a diameter of 0.15 mm and smaller than approx 150 mN for a diameter of 0.25 mm.

Some examples follow of mesh-like basic structures of which versions of the implant according to the invention consist. In all cases the basic structures are made as crochet galloon ware from "Pronova" monofilaments.

### Example 1

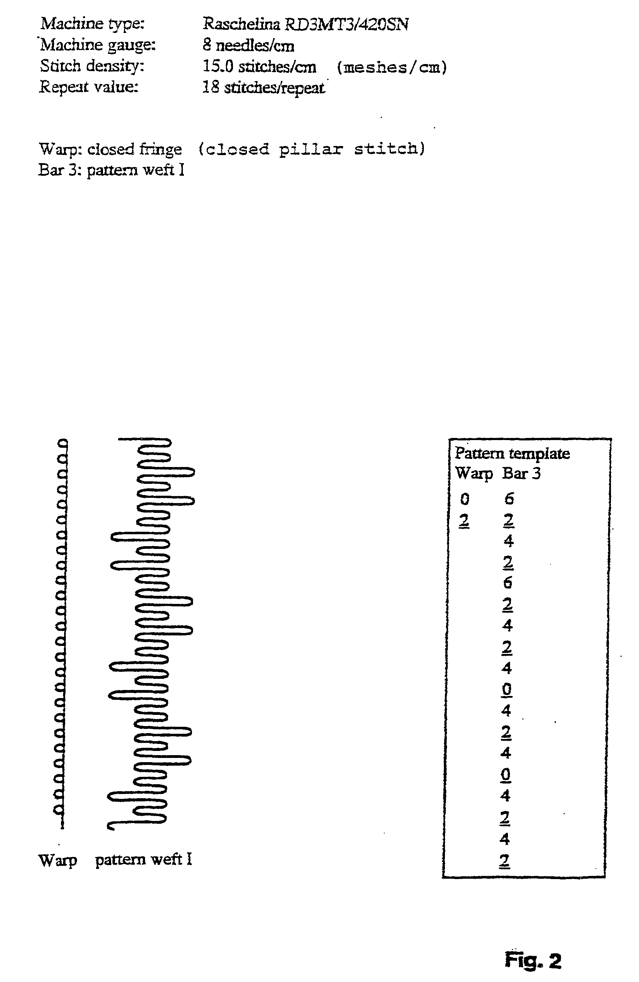

A monofilament of "Pronova" (see above), with a mixture ratio of the homopolymer to the copolymer of 80/20 (in wt.-%) and with a diameter of 0.079 mm, was worked into crochet galloon ware on a "Raschelina RD3MT3/420SN" type crochet galloon machine. The production specification as well as the pattern template emerge from Figure 2 in a way familiar to the person skilled in the art.

After being warp-knitted, the mesh-like basic structure was washed and subsequently stretched on a frame and thermofixed at temperatures between 80°C and 130°C. Afterwards the mesh-like basic structure was cut to size as a flexible implant, packed and subjected to a gas sterilisation with ethylene oxide.

### Example 2

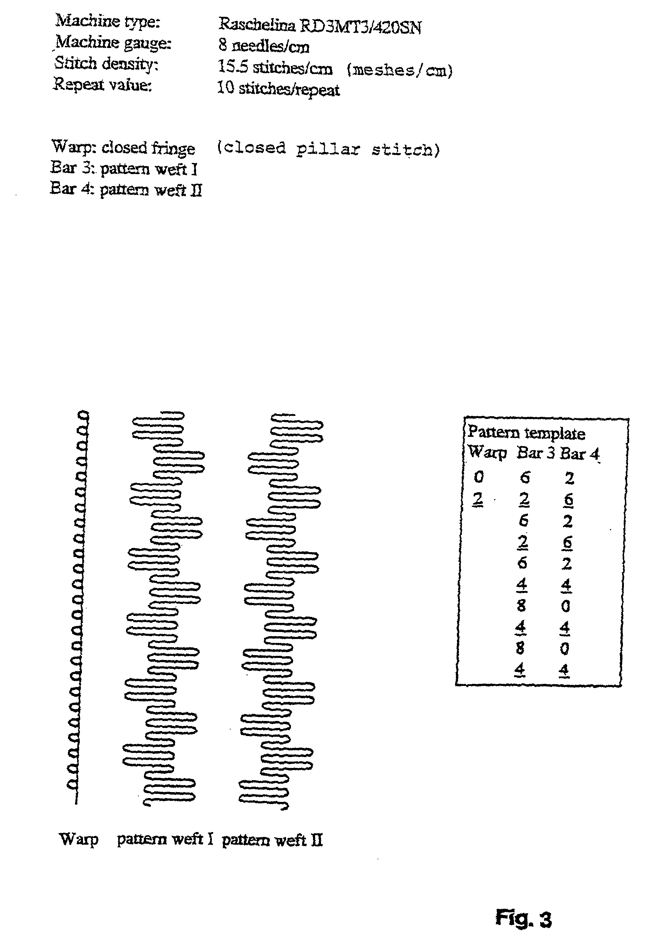

Crochet galloon ware was made from a "Pronova" monofilament (see above) of 0.079 mm diameter with a mixture ratio of homopolymer to copolymer of 80/20 (in wt.-%) similarly to Example 1. The production specification as well as the pattern template emerge from Figure 3.

Subsequently the mesh-like basic structure was washed as in Example 1, thermofixed, packed as a flexible implant and sterilized.

An implant produced in this way was inserted intraperitoneally in the abdominal wall of a rabbit and secured with clips (so-called IPOM-technique, "Intraperitoneal Onlay Mesh"). For comparison, a commercial implant mesh of polypropylene ("Marlex", see below) was implanted in an analogous way. Directly after the surgery the implant according to Example 2 lay flat, and the edges had not rolled up. On the other hand, the implant of polypropylene was lying incorrectly, the edges having slightly rolled up and sticking out from the bottom layer.

Seven days later, the conventional implant mesh had caused very marked adhesions over the whole surface, which was attributable inter alia to a mechanical irritation of the peritoneum. On the other hand, the implant according to the invention was covered by a thin, transparent, smooth mesothelium cell layer. Surprisingly, there were no adhesions.

### Example 3

A mesh-like basic structure was warp-knitted as crochet galloon ware as in Example 2 and further processed, the only difference being that in this case a "Pronova" monofilament (see above) with a diameter of 0.093 mm and a mixture ratio of homopolymer to copolymer of 50/50 (in wt.-%) was used. The production specification and the pattern template again result from Figure 3.

### Example 4

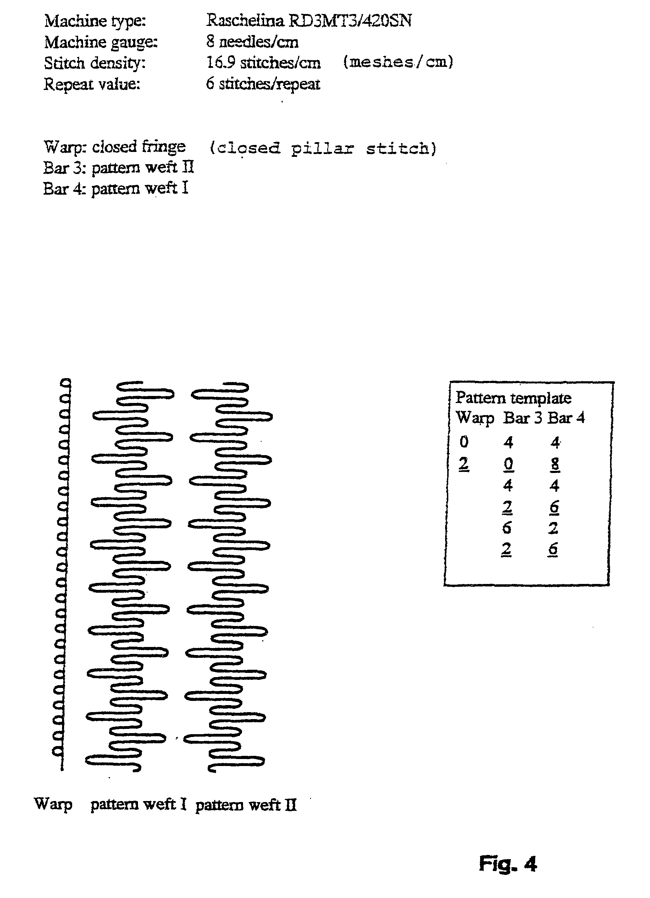

Crochet galloon ware was produced in a similar way to Example 1 from a "Pronova" monofilament (see above) of 0.079 mm diameter with a mixture ratio of homopolymer to copolymer of 80/20 (in wt.-%). The production specification as well as the pattern template result from Figure 4.

Subsequently, the mesh-like basic structure was washed as in Example 1, thermofixed, packed as a flexible implant and sterilized.

### Example 5

A mesh-like basic structure was warp-knitted as crochet galloon ware as in Example 4 and further processed, the only difference being that in this case "Pronova" monofilament (see above) with a diameter of 0.093 mm and a mixture ratio of homopolymer to copolymer of 50/50 (in wt.-%) was used. The production specification and the pattern template again result from Figure 4.

The approximate pore size, the bending force, the bending rigidity as well as the bending elasticity modulus are compiled in Table 4 for the five implants according to the invention according to Examples 1 to 5 as well as for two conventional implants of polypropylene ("Marlex" and "Atrium"). "Marlex" is a trade name for implant meshes marketed by C.R. Bard Inc.; "Atrium" is the name given to implant meshes marketed by Atrium Medical Corporation.

The pore size is the greatest pore width of the mesh-like basic structure and was determined with a measuring magnifier.

The bending force and the bending rigidity were measured using three-point bending tests, as explained above. These variables were normalized to a sample width of 10 mm. The bending elasticity modulus is calculated from the shape of the curve, taking into account the thickness and width of the sample.

It will be seen that the bending force and the bending rigidity correlating with the bending force are clearly lower in the case of the versions of the implant according to the invention than in the case of the conventional implants. On the other hand, the pores are smaller in the case of the conventional implants.

## Claims

1. Flexible implant, with a mesh-like basic structure of monofilaments, the diameter of which is in the range from 0.02 mm to 0.30 mm and the bending elasticity modulus of which is smaller than 3500 N/mm², the basic structure containing pores the size of which lies in the range from 1.5 mm to 8.0 mm over more than 90% of the total area of the pores, and the bending force of the basic structure, measured in a three-point bending test with a span of 11 mm and normalized to a sample width of 10 mm, being 30 mN at most.

2. Implant according to claim 1, **characterized in that** the basic structure contains monofilaments of at least one of the following materials: thermoplastically workable fluorinated homopolymers, thermoplastically workable fluorinated copolymers, polyolefins, mixtures of the aforementioned materials.

3. Implant according to claim 2, **characterized in that** the basic structure contains monofilaments of at least one of the following materials: polyvinylidene fluoride, copolymers of vinylidene fluoride and hexafluoropropene, mixtures of the aforementioned materials.

4. Implant according to claim 3, **characterized in that** the basic structure contains monofilaments of at least one of the following materials: mixtures of polyvinylidene fluoride and a copolymer of 95 wt.-% vinylidene fluoride and 5 wt.-% hexafluoropropene, preferably in the mixture ratio 50/50 or 80/20.

5. Implant according to one of claims 1 to 4, **characterized in that** the basic structure contains monofilaments of a resorbable material.

6. Implant according to claim 5, **characterized in that** the basic structure contains monofilaments of at least one of the following materials: poly-p-dioxanone, copolymers of glycolides and caprolactones.

7. Implant according to one of claims 1 to 6, **characterized in that** the tensile strength of at least part of the monofilaments is at least 400 N/mm².

8. Implant according to one of claims 1 to 7, **characterized in that** the basic structure contains a weft-knitted product or a warp-knitted product, preferably a warp-knitted product made as crochet galloon ware.

## Patentansprüche

1. Flexibles Implantat, mit einer netzartigen Grundstruktur aus Monofilamenten, deren Durchmesser im Bereich von 0,02 mm bis 0,30 mm liegt, und deren Biegeelastizitätsmodul kleiner als - 3500 N/mm² ist, wobei die Grundstruktur Poren aufweist, deren Größe über mehr als 90% der Gesamtfläche der Poren im Bereich von 1,5 mm bis 8,0 mm liegt und wobei die Biegekraft der Grundstruktur, gemessen in einem Dreipunkt-Biegeversuch bei einer Stützweite von 11 mm und normiert auf eine Probenbreite von 10 mm, höchstens 30 mN beträgt.

2. Implantat nach Anspruch 1, **dadurch gekennzeichnet, dass** die Grundstruktur Monofilamente aus mindestens einem der folgenden Materialien aufweist: thermoplastisch verarbeitbare fluorhaltige Homopolymere, thermoplastisch verarbeitbare fluorhaltige Copolymere, Polyolefine, Mischungen der vorgenannten Materialien.

3. Implantat nach Anspruch 2, **dadurch gekennzeichnet, dass** die Grundstruktur Monofilamente aus mindestens einem der folgenden Materialien aufweist: Polyvinylidenfluorid, Copolymere aus Vinylidenfluorid und Hexafluorpropen, Mischungen der vorgenannten Materialien.

4. Implantat nach Anspruch 3, **dadurch gekennzeichnet, dass** die Grundstruktur Monofilamente aus mindestens einem der folgenden Materialien aufweist: Mischungen aus Polyvinylidenfluorid und einem Copolymer aus 95 Gew.-% Vinylidenfluorid und 5 Gew.-% Hexafluorpropen, vorzugsweise im Mischungsverhältnis 50/50 oder 80/20.

5. Implantat nach einem der Ansprüche 1 bis 4, **dadurch gekennzeichnet, dass** die Grundstruktur Monofilamente aus einem resorbierbaren Material aufweist.

6. Implantat nach Anspruch 5, **dadurch gekennzeichnet, dass** die Grundstruktur Monofilamente aus mindestens einem der folgenden Materialien aufweist: Poly-p-dioxanon, Copolymere aus Glykoliden und Caprolactonen.

7. Implantat nach einem der Ansprüche 1 bis 6, **dadurch gekennzeichnet, dass** die Reissfestigkeit zumindest eines Teils der Monofilamente mindestens 400.N/mm² beträgt.

8. Implantat nach einem der Ansprüche 1 bis 7, **dadurch gekennzeichnet, dass** die Grundstruktur ein Gestrick oder Gewirke aufweist, vorzugsweise ein als Häkelgalonware ausgeführtes Gewirke.

## Revendications

1. Implant flexible, doté d'une structure de base de type treillis constituée de monofilaments dont le diamètre est dans le domaine de 0,02 mm à 0,30 mm et dont le module d'élasticité de courbure est inférieur à 3 500 N/mm², la structure de base contenant des pores dont la taille est dans le domaine de 1,5 mm à 8,0 mm sur plus de 90 % de la surface totale des pores, et la force de courbure de la structure de base, mesurée au cours d'un test de courbure en trois points avec une étendue de 11 mm et normalisée à une largeur échantillon de 10 mm, étant de 30 mN au maximum.

2. Implant selon la revendication 1, **caractérisé en ce que** la structure de base contient des monofilaments d'au moins l'un des matériaux suivants : homopolymères fluorés usinables par procédés thermoplastiques, polyoléfines, mélanges des matériaux mentionnés ci-dessus.

3. Implant selon la revendication 2, **caractérisé en ce que** la structure de base contient des monofilaments d'au moins l'un des matériaux suivants : polyfluorure de vinylidène, copolymères de fluorure de vinylidène et hexafluoropropène, mélange des matériaux mentionnés ci-dessus.

4. Implant selon la revendication 3, **caractérisé en ce que** la structure de base contient des monofilaments d'au moins l'un des matériaux suivants : mélanges de polyfluorure de vinylidène et d'un copolymère de 95 % en poids de fluorure de vinylidène et de 5 % en poids d'hexafluoropropène, de préférence avec un ratio de mélange de 50/50 ou de 80/20.

5. Implant selon l'une quelconque des revendications 1 à 4, **caractérisé en ce que** la structure de base contient des monofilaments en matériau résorbable.

6. Implant selon la revendication 5, **caractérisé en ce que** la structure de base contient des monofilaments d'au moins l'un des matériaux suivants : poly-p-dioxanone, copolymères de glycolides et de caprolactones.

7. Implant selon l'une des revendications 1 à 6, **caractérisé en ce que** la résistance à la traction d'au moins une partie des monofilaments est d'au moins 400 N/mm².

8. Implant selon l'une des revendications 1 à 7, **caractérisé en ce que** la structure de base contient un produit à mailles cueillies ou un produit tricoté en chaîne, de préférence un produit tricoté en chaîne fabriqué comme un galon crocheté.
